# EUROPEAN PATENT APPLICATION

(11) **EP 1 512 970 A1**
(43) Date of publication of application: **09.03.2005**
(21) Application number: 03077804.7
(22) Date of filing: 05.09.2003
(51) Int. Cl.: G01N 33/50

(54) **Method for determining the impact of a multicomponent mixture on the biological profile of a disease**

(71) Applicant: Nederlandse Organisatie voor Toegepast-Natuurwetenschappelijk Onderzoek TNO, 2628 VK Delft (NL)
(72) Inventor: Wang, Mei, 2341 BV Oegstgeest (NL); Van der Greef, Jan, 3971 BM Driebergen (NL); Witkamp, Renger, 3662 LM Wijk bij Duurstede (NL)
(74) Representative: Prins, Adrianus Willem, Mr. Ir.

(57) **Abstract**

The present invention provides a method for determining the impact of a multicomponent mixture on a biological profile of a disease comprising the steps of:
(a) determining a biological profile of the disease by comparing the biological profile of a group with symptoms of the disease with the biological profile of a reference (or healthy) group, using a multivariate analysis;
(b) determining the impact of a series of samples of the multicomponent mixture on the biological profile of the disease, in which respective samples the concentrations of various components differ, using a multivariate analysis;
(c) determining the composition of the samples of the multicomponent mixture that have shown in step (b) an impact on the biological profile of the disease, using a multivariate analysis;
(d) identifying within the compositions as determined in step (c) the effective components and their respective concentrations required for having an impact on the biological profile of the disease, using a multivariate analysis.

The invention also provides a method for preparing a medicament for treating a disease, wherein the effective components as identified in step (d) are combined in the respective concentrations required for having an impact on the biological profile of the disease. The invention further provides a method for controlling the composition of a multicomponent mixture, wherein the concentrations of at least two components of the mixture are adjusted to ensure that the at least two components of the mixture have an impact on a biological profile of the disease.

## Description

The present invention is related to the field of life sciences and the domain of health and diseases, in particular the development of strategies and products for the prevention, treatment or curing of a disease.

In contrast with the reductionistic approach as conventionally applied by pharmaceutical companies to develop new medicaments, the present invention is based on a holistic approach to living organisms.

Such a holistic approach to living organisms has, for instance, been the basis of applying herbal medicine such as Traditional Chinese Medicine (TCM) from its earliest years. An important starting point in herbal medicine based approaches is that every healthy organism is in balance. Balance is considered to be a complex interplay between body and mind, which is reflected at all levels ranging from the biochemical component perspective to the energetic system control of our physical body. Internal imbalances can stem from a wide variety of factors and lead to a plethora of conditions ranging from short perturbations to chronic disease processes. Additionally, in such an approach as in TCM the uniqueness of each human being is recognized and drives the necessity to develop a personalized medication to obtain optimal results based on multi-component treatments.

At a first glance the "Western" medical approach may seem very different from this. However, the revolution in genomics that has taken place in life sciences during the last decade has provided considerable support for a more holistic view on diagnosis and treatment. Furthermore, the issue of personalized medicine is now receiving considerable attention due to the new insights in i.a. pharmacogenomics. Although the principle of homeostasis has been a cornerstone of Western physiology for more than a century, the enormous complexity of biological systems has often driven pharmaceutical research towards trying to identify and influence only single targets that make the difference between health and disease. This approach has indeed yielded many potent drugs but also revealed major drawbacks. In fact one tries to influence a system by interacting with a single protein that is often part of a complex pathway and involved in a cascade of reactions and feed-back loops. The reality is that most diseases are multi-factorial which means that treating a single target provides a partial treatment (reduction of symptoms) and in the majority of cases no cure. Although this awareness is not new, it has been impossible to find alternative routes giving the mentioned complexity of the system.

A method has, however, now been developed that enables highly detailed profiling of complex mixtures and subsequent measurements of the complex multicomponent induced changes in biological systems (biological effects) such as *in-vitro* (such as cell cultures) and *in-vivo* systems (such as animal models, humans). In this method the interaction of multiple components in complex mixtures such as natural products or extracts and mixtures thereof (including nutraceutical products, functional food products, herbal medicinal products, other natural compounds and biofluids) with living biological systems can very effectively be measured, using a particular set of steps wherein technologies are applied such as biostatistics and bioinformatics. By means of such measurements the impact of multicomponent mixtures on the biological profile of a disease can advantageously be determined. Moreover, such measurements enable the identification of the effective components within the multicomponent mixtures and their respective concentrations required for having an impact on the biological profile of the disease can be identified.

Accordingly, the present invention relates to a method for determining the impact of a multicomponent mixture on a biological profile of a disease comprising the steps of:
(a) determining a biological profile of the disease by comparing the biological profile of a group with symptoms of the disease with the biological profile of a reference (or healthy) group, using a multivariate analysis;
(b) determining the impact of a series of samples of the multicomponent mixture on the biological profile of the disease, in which respective samples the concentrations of various components differ, using a multivariate analysis;
(c) determining the composition of the samples of the multicomponent mixture that have shown in step (b) an impact on the biological profile of the disease, using a multivariate analysis;
(d) identifying within the compositions as determined in step (c) the effective components and their respective concentrations required for having an impact on the biological profile of the disease, using a multivariate analysis.

The method according to the present invention enables the measurement of the effects of multiple target interventions and the development of products to optimally perform such interventions by a unique approach, revealing the biological profile of the effective components. The present invention provides, therefore, an important bridge function between the two complementary approaches used in TCM and Western Medicine, because it can reveal besides the effects of simple perturbations like a single drug also the complex perturbations using multicomponent mixtures such as, for instance, herbal medicine products and functional food products. This unique approach is referred to as multidimensional pharmacology (MDP) and uses the Systems Biology approach in which biological systems are studied by measuring and integrating metabolic data and other profile data, such as genetic and/or proteomic data.

The determination of biological effects and in particular synergetic multicomponent effects in accordance with the present invention is illustrated by the example of herbal medicine products in intervention strategies. The determination of such effects is not limited to biological and synergetic effects in mammalian systems but can address all possible forms of living systems with complex mixtures derived from the same portfolio of life.

The present invention is not limited to a particular type of disease, but embraces any disease of which a biological profile can be determined.

A considerable advantage of the present invention resides in the fact that it provides a scientific basis for both efficacy and safety of the tested multicomponent mixtures.

The method of the present invention allows characterization of the complex multicomponent mixtures, in the case of TCM-products, the effects of these products in mammalian systems to reveal biological effects and the effects of TCM on patients.

A further very important aspect of the method according to the present invention is that it allows the measurement of all biological effects including additive and synergetic effects.

In health and disease studies a bodyfluid profile of for instance a plasma sample from a control group (reference group) and patient group ( a group with symptoms of the disease with the biological profile) can be used to measure as many components as possible and is evaluated for differences in single components or patterns of components between the two groups to obtain a better insight in the underlying biological mechanisms, to detect novel biomarkers/surrogate markers, to predict toxicology or pharmacological response or to develop novel intervention routes. In the context of the present application a biomarker is defined as a characteristic that is objectively measured and evaluated as an indicator of: normal biological processes, pathogenic processes or pharmacological response to a therapeutic intervention. Biomarkers can be genes, transcripts, proteins, metabolites, (trace)-elements or any combination of those elements.

The concept of the present invention of using patterns for dynamic modeling directly or after non-linear or linear multidimensional compression opens the unique route of studying dynamical processes using systems descriptions based on component patterns. In such studies perturbations as for instance via drug intervention routes can be monitored and evaluated in a multidimensional way and methods such as time series analysis, time warping and non-linear dynamic techniques can be applied. In the evaluation of data generated with the method of the present invention also the addition of other data and information from other sources can be of importance such as information from clinical dossiers of patients describing the medical diagnosis and clinical chemistry, or disease studies data on behavior, cognition, psychological, social, etc., levels. These data can be included or linked to the data set created by the method according to the present invention to classify patients or to discover sub-classes or other relevant observations.

The application of the present invention creates a wide variety of novel approaches in using metabolomics and/or other component profiles of bodyfluids to enhance the overall process of biomedicine or drug discovery, development including clinical evaluation, diagnostic applications and post-marketing surveillance. The created profiles can be used first of all to obtain a better insight in the underlying biological process a.o. for toxicology evaluation (predictive toxicology), biomarker/surrogate marker or biomarker/surrogate marker pattern discovery, protein target validation, comparison of animal models and relating these to human studies, phenotyping at the metabolite level, responder/non-responder evaluation, validation of animal models such as transgenic models, providing the ability to correlate metabolite level data with other levels in systems biology such as gene, mRNA, RNAi and protein data.

The method according to the present invention generates patterns of components and the used multivariate analysis generates patterns of relevant components for a given situation or investigation. In modern biology and related nutraceutical, pharmaceutical and biotechnological industries this is a crucial new paradigm for driving the scientific research and industrial discovery and development processes. The basic understanding is that biology in general and disease processes in particular are multifactorial of nature and therefore the understanding of such processes requires a description or understanding based on a multitude of components. In most cases development processes are for instance based on a single target evaluated with a single biomarker for efficacy or for differentiating of control groups from patients groups. The present invention, however, provides a method that allows the creation of a breakthrough in the ability to describe multifactorial diseases by multifactorial patterns as well as multifactorial responses toward multifactorial input variables such as in combination therapies (chemotherapy) or in evaluation of herbal medicine, functional food and nutraceutical responses.

In accordance with the present invention preferably at least one spectrometric technique or at least one chromatographic technique is used in step (a) to determine the profile of the disease. More preferably, use is made of two or more spectrometric techniques. Most preferably, use is made of at least a nuclear magnetic resonance technique and a mass spectrometry technique to determine the profile of the disease in step (a).

The biological profile to be determined in step (a) includes preferably one or more metabolic, genetic and/or proteomic profiles. Any combination of these profiles can be used. Preferably, such combination includes one or more metabolic profiles. More preferably, the biological profile includes metabolic, genetic and proteomic profiles.

In step (a) preferably the biological profile is determined of at least one type of bodyfluid or at least one type of tissue. More preferably, in step (a) the biological profiles are determined of at least two different types of bodyfluid.

The biological profiles are determined in step (a) using one or more of the following biomarkers; genes, transcripts, proteins, metabolites and (trace) elements.

In step (b) preferably use is made of at least one spectrometric technique or at least one chromatographic technique to determine the impact of the series of samples of the multicomponent mixture on the biological profile of the disease. More preferably, use is made of two or more spectrometric techniques, whereas most preferably use is made of at least a nuclear magnetic resonance technique and a mass spectrometry technique.

In step (c) preferably use is made of at least one spectrometric technique or at least one chromatographic technique to determine the composition of the samples. More preferably, use is made of two or more spectrometric techniques, whereas most preferably use is made of at least a nuclear magnetic resonance technique and a mass spectrometry technique. In step (c) the number of samples of which the composition is determined in (c) is preferably at least 2, and more preferably in the range of 5-100.

In step (d) preferably use is made of at least one spectrometric technique or at least one chromatographic technique to identify the effective components and their respective concentrations required for having an impact on the biological profile of the disease. More preferably, use is made of two or more spectrometric techniques. Most preferably use is made of at least a nuclear magnetic resonance technique and a mass spectrometry technique in step (d).

The multicomponent mixture to be used in accordance with the present invention may be any multicomponent mixture having a (potential) impact on a biological profile of a disease. Such multicomponent mixture may be a synthetic product or a natural product. Suitable examples of such mixture include nutraceutical products, functional food products, chemical products, herbal medicinal products or biofluids.

In each of steps (a)-(d) preferably use of at least one spectrometric technique. Suitably a nuclear magnetic resonance technique ("NMR") or mass spectrometry technique ("MS") can be used, whereby the latter technique focuses on a limited number of small molecule compounds. Both of these techniques have however limitations. The NMR approaches are limited in that they typically provide reliable information only of compounds present at high concentration. On the other hand, focused mass spectrometry techniques do not require high concentrations but can provide information of only limited portions of a biological profile. As used herein, the terms "small molecule" and "metabolite" are used interchangeably. Small molecules and metabolites include, but are not limited to, lipids, steroids, amino acids, organic acids, bile acids, eicosanoids, peptides, and trace elements.

Therefore, in each of steps (a)-(d) preferably use is made of at least a nuclear magnetic resonance technique and a mass spectrometry technique.

Sample preparation for NMR can generally be very straightforward using freeze-drying and reconstitution in D₂O because the focus is on the higher concentration components, i.e. of typical concentration > 100 nanogram/mL. For MS a variety of sample preparation approaches can be used ranging from solid phase extraction and liquid/liquid extraction to more specific methods using, for instance, affinity-based methods or derivitization procedures both for GC-MS as well as LC-MS.

In each of steps (a)-(d) use can be made of spectrometric data obtained from one or more platforms including, but not limited to, MS, NMR, liquid chromatography ("LC"), gas-chromatography ("GC"), high performance liquid chromatography ("HPLC"), capillary electrophoresis ("CE"), and any known form of hyphenated mass spectrometry in low or high resolution mode, such as LC-MS, GC-MS, CE-MS, LC-UV, MS-MS, MSⁿ, etc.

As used herein, the term "spectrometric data" includes data from any spectrometric or chromatographic technique. Spectrometric techniques include, but are not limited to, resonance spectroscopy, mass spectroscopy, and optical spectroscopy. Chromatographic techniques include, but are not limited to, liquid phase chromatography, gas phase chromatography, and electrophoresis.

If a spectral profile is obtained, as with standard spectroscopic methods, a primary necessary step is to adjust for minor shifts in the spectra both in the intensity dimension as well as in the spectral or chromatographic dimension. Shifts can be due to instrumental factors, environmental conditions, or to varying concentrations of components (as is often the case in urine analysis). As an example, variation in NMR chemical shifts often occurs and needs to be accounted for, but the reproducibility and standardization in the intensity (or peak area) of a single profile (quantitation dimension) is typically very satisfactory. This is in contrast to MS where the peak intensity (ion abundance) dimension needs to be carefully adjusted or standardized due to lack of calibrants for each component present in the profile. In hyphenated techniques the reproducibility of the separation method (GC, LC or electromigration driven techniques such as capillary electrophoresis (CE)) needs to be carefully evaluated as well. In this respect near-infrared spectral profiles are impressive and correction in either dimension is hardly required.
In general, small instrumental shifts in the spectral (variable) dimension will be falsely interpreted as representing different components when a collection of data profiles is subjected to pattern recognition analysis. A straightforward way to cope with this problem is by using binning techniques in which the spectrum is reduced in resolution to a sufficient degree to insure that a given peak remains its bin despite small spectral shifts between analyses. For example, in NMR the chemical shift axis may be descretized and coarsely binned, and in MS the spectral accuracies may be rounded to integer atomic mass unit values. However, more subtle procedures are preferred such as partial linear fit for NMR or other alignment procedures for MS.

After initial data pre-processing the spectral profiles are set for pattern recognition (multivariate analysis).

The ability to use different techniques to produce bodyfluid profiles is optimally used by multiway multivariate analysis and allows the measurement of different bodyfluids of the same system (such as plasma and urine or plasma and CSF) to reveal novel insights into the systems biology, for instance the effect of the blood brain barrier when comparing plasma and urine profiles. Hence, in each of steps (a)-(d) preferably use is made of multiway multivariate analysis.

The present invention provides a method of spectrometric data processing utilizing multiple steps of multivariate analysis to process data in a hierarchal procedure (steps (a)-(d)). In each of steps (a)-(d) a first multivariate analysis can be used on a plurality of data sets to discern one or more sets of differences and/or similarities between them, whereafter a second multivariate analysis can be used to determine a correlation (and/or anti-correlation, i.e., negative correlation) between at least one of these sets of differences (or similarities) and one or more of the plurality of data sets. In step (a) the determination of the biological profile of a disease may also be based on the correlation.

As used herein, the term "data sets" refers to the spectrometric data associated with one or more spectrometric measurements. For example, where the spectrometric technique is NMR, a data set may comprise one or more NMR spectra. Where the spectrometric technique is UV spectroscopy, a data set may comprise one or more UV emission or absorption spectra. Similarly, where the spectrometric technique is MS, a data set may comprise one or more mass spectra. Where the spectrometric technique is a chromatographic-MS technique (e.g., LC-MS, GC-MS, etc), a data set may comprise one or more mass chromatograms. Alternatively, a data set of a chromatograms or reconstructed TIC chromatograms. In addition, it should be realized that the term "data sets" includes both raw spectrometric data and data that has been pre-processed (e.g., to remove noise, baseline, detect peaks, etc.).

Moreover, as used herein, the term "data sets" may refer to substantially all or a sub-set of the spectrometric data associated with one or more spectrometric measurements. For example, the data associated with the spectrometric measurements of different sample sources (samples of groups with symptoms of the disease (experimental group samples) v. samples of reference or healthy groups (control group samples)) may be grouped into different data sets. As a result, a first data set may refer to experimental group sample measurements and a second data set may refer to control group sample measurements. In addition, data sets may refer to data grouped based on any other classification considered relevant.

The present invention also provides a method of spectrometric data processing utilizing multivariate analysis to process data at two or more hierarchal levels of correlation. In each of steps (a)-(d) use can be made of a multivariate analysis on a plurality of data sets to discern correlations (and/or anti-correlations) between data sets at a first level of correlation, whereafter the multivariate analysis can be used to discern correlations (and/or anti-correlations) between data sets at a second level of correlation. In step (a) the determination of the biological profile of a biological system may also be based on the correlations discerned at one or more levels of correlation.

In accordance with the present invention the spectrometric data processing in each of steps (a)-(d) can be carried out utilizing multiple steps of multivariate analysis to process data sets in a hierarchal procedure, whereby one or more of the multivariate analysis steps further comprises processing data at two or more hierarchal levels of correlation. For example, in each of steps (a)-(d) a first multivariate analysis can be used on a plurality of data sets to discern one or more sets of differences and/or similarities between them; a second multivariate analysis can be used to determine a first level of correlation (and/or anti-correlation) between a first set of differences (or similarities) and one or more of the data sets; and the second multivariate analysis can be used to determine a second level of correlation (and/or anti-correlation) between the first set of differences (or similarities) and one or more of the data sets. In step (a) the determination of the biological profile of the disease can be based on the correlations discerned at one or more levels of correlation.

Suitable forms of multivariate analysis include, for example, principal component analysis ("PCA"), discriminant analysis ("DA"), PCA-DA, canonical correlation ("CC"), partial least squares ("PLS"), predictive linear discriminant analysis ("PLDA"), neural networks, multiway/multiblock analysis, support vector machines ("SVM"), parafac and pattern recognition techniques.

The use of the above-described multivariate analysis techniques is well-known in the art. For a more detailed description reference can, for instance, be made to US Patent Application Serial No. 10/218,880 (Method and System for Profiling Biological Systems) of which the entire contents are hereby incorporated by reference. To extract the maximum value from the data, multivariate analysis tools as outlined above may be used in concert with additional statistical and informatics strategies. Once statistically significant differences in, for instance, metabolite abundances are determined and quantified among groups of samples, the objective becomes to understand the underlying biological reasons for and the contexts of the results. A first step is to identify metabolic components observed in data spectra and revealed by multivariate analysis to be significant differences among samples. Such identification typically involves querying various databases of known metabolite component spectra and structures. A next step is to mine existing knowledge about molecular interactions through searches of public and private databases. This can go some way in explaining the associations and behaviour observed in the metabolomic profile results. However, because most of the metabolic, genomic, proteomic, and interaction databases depict biochemical events in a static state, progressively sophisticated analytical and mathematical tools are needed to integrate disjointed biological clues into dynamic models that are better suited to explain, for example, pathological processes.

Indeed, both linear and non-linear multivariate analyses may uncover statistically significant associations between biomolecular components that will not be explained through the mining of existing databases or literature.

A preferred embodiment of the method of the present invention comprises the following steps :
1. A selection is made of the relevant samples, for instance bodyfluids (plasma, urine, CSF, saliva, synovial fluid etc.)
2. A selection is made of the width of the biological profiling; transcripts, proteins, metabolites, etc.
3. A sample is prepared based on the spectrometric techniques to be used for determining the biological profile (e.g. GCMS, LCMS, CEMS, MS/MS combinations and various NMR-methodologies, etc.)
4. The profile is determined using the spectrometric techniques, NMR-profiles and preferred MS-approaches in case of metabolomics lipids, steroids, bile acids, eicosanoids, (neuro)peptides, vitamins, organic acids, neurotransmitters, amino acids, carbohydrates, ionic organics, nucleosides, inorganics, xenobiotics, etc., with a preference to include peptides. Also a global MS-profile to describe in a single experiment the major high concentration components can be included which is often a good indication for the balance/homeostasis of a system in addition to the NMR-profile.
5. The data obtained is preprocessed using preferable the technique described in Dutch patent application 1016034, in combination with PCA-DA, multiblock/multiway multivariate analysis based on linear and non-linear techniques and the partial linear fit algorithm.
6. The outcome of 4 is combined with other relevant data sources such as medical history, clinical chemistry records, medical description and behavioural, social, psychological data, etc.
7. The (non-linear or linear) dynamics of dynamical diseases are studied by using one of the profiling-components or any combination of the profiling components, preferably by using a combination of non-linear compression and dynamic modeling techniques.

The concept of the present invention is suitably based on the following aspects, the profiling of complex mixtures such as body fluids by a combination of NMR and a selection of hyphenated mass spectrometric techniques (GC-,LC-,-CE-MS/MS, ICPMS), the evaluation of the combination with data preprocessing/scaling preceding multiblock/multiway multivariate analysis, the combination of instrumental datasets created with other relevant datasets, the linking of data sets arising from samples from one system but via different bodyfluid profiles and the ability to study all forms of non-linear dynamics.

In cases where all biological active components are detected and the composition constraints for optimal effect are revealed, the knowledge can be used to design mixtures of synthetically obtained components. In the latter case pharmaceutical preparations can be generated.

Hence, the present invention also relates to a method for preparing a medicament for treating a disease, wherein the effective components as identified in step (d) are combined in the respective concentrations required for having an impact on the biological profile of the disease. Suitably, the effective components are synthetically prepared.

The detection of biological active patterns in mixtures such as herbal products enables also the focused quality control or optimized production of plants enabling efficient and well controlled products. Therefore, the present invention further relates to a method for controlling the composition of a multicomponent mixture, wherein the concentrations of at least two components of the mixture are adjusted to ensure that the at least two components of the mixture have an impact on a biological profile of the disease. The multicomponent mixture can for instance be contained in a plant which quality is controlled and adjusted to produce a high quality product. Thus, the multicomponent mixture may comprise a natural product.

The detection of biological active patterns in mixtures such as herbal products enables also the focused quality control or optimized production of plants enabling efficient and well controlled products.

In cases where all or part of the biological active components are detected and the composition constraints for optimal effect are revealed, the knowledge can be used to design mixtures of synthetically obtained components. In the latter case pharmaceutical preparations can be generated.

### Example

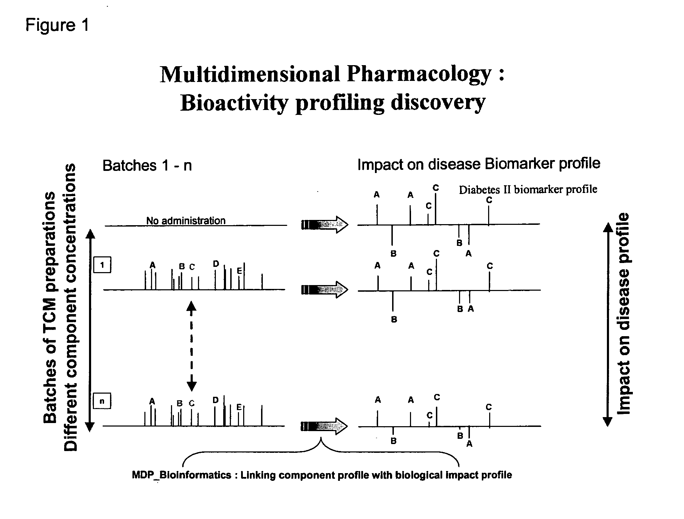

The method according to the present invention is schematically depicted in Figure 1. A typical experiment in accordance with the present invention based on the fingerprints as generated by the systems biology approach a typical experiment is based on the following steps :
1. A number of batches of a mixture, preferably with a significant variation in composition, is measured (fingerprinted) by a profiling technique such as NMR or mass spectrometry ; indicated as batches 1-n in the left part of Figure 1.
2. The profiles of the effect of those batches after administration into an animal model or in a human trial are measured as well as a reference group which is not treated (comprised of both disease animals and wildtype or healthy subjects/patients) or chosen to be treated in another way.
3. The reference group provides the biomarker profile for the disease and the other experiments provide the impact of the mixture on the disease pattern and reveals also other effects. This yields a prove of effect on the specific disease group.
4. (Non)-linear multivariate correlation of the patterns of the mixture components and the effect profiles enables the detection of the patterns of components responsible for the biological effects.

## Claims

1. Method for determining the impact of a multicomponent mixture on a biological profile of a disease comprising the steps of:
(a) determining a biological profile of the disease by comparing the biological profile of a group with symptoms of the disease with the biological profile of a reference (or healthy) group, using a multivariate analysis;
(b) determining the impact of a series of samples of the multicomponent mixture on the biological profile of the disease, in which respective samples the concentrations of various components differ, using a multivariate analysis;
(c) determining the composition of the samples of the multicomponent mixture that have shown in step (b) an impact on the biological profile of the disease, using a multivariate analysis;
(d) identifying within the compositions as determined in step (c) the effective components and their respective concentrations required for having an impact on the biological profile of the disease, using a multivariate analysis.

2. Method according to claim 1, wherein in (a) use is made of at least one spectrometric technique or at least one chromatographic technique to determine the profile of the disease.

3. Method according to claim 1 or 2, wherein in (b) use is made of at least one spectrometric technique or at least one chromatographic technique to determine the impact of the series of samples of the multicomponent mixture on the biological profile of the disease. samples.

4. Method according to any one of claims 1-3, wherein in (c) use is made of at least one spectrometric technique or at least one chromatographic technique to determine the composition of the samples.

5. Method according to any one of claims 1-4, wherein in (d) use is made of at least one spectrometric technique or at least one chromatographic technique to identify the effective components and their respective concentrations required for having an impact on the biological profile of the disease.

6. Method according to any one of claims 2-5, wherein use is made of two or more spectrometric techniques.

7. Method according to claim 6, wherein use is made of at least a nuclear magnetic resonance technique and a mass spectrometry technique.

8. Method according to any one of claims 1-7, wherein the biological profile includes one or more metabolic, genetic and/or proteomic profiles.

9. Method according to claim 8, wherein the biological profile includes the metabolic, genetic and proteomic profiles.

10. Method according to any one of claims 1-9, wherein the multicomponent mixture comprises a nutraceutical product, functional food product, chemical product, herbal medicinal product or biofluid.

11. Method according to any one of claims 1-10, wherein in (a) the biological profiles are determined of at least one type of bodyfluid.

12. Method according to any one of claims 1-11, wherein in (a) the biological profiles are determined of at least one type of tissue.

13. Method according according to claim 11, wherein in (a) the biological profiles are determined of at least two different types of bodyfluid.

14. Method according to any one of claims 1-13, wherein in (a) the biological profiles are determined using one or more of the following biomarkers; genes, transcripts, proteins, metabolites and (trace) elements.

15. Method according to any one of claims 1-14, wherein the number of samples of which the composition is determined in (c) is at least 2.

16. Method according to claim 15, wherein the number of samples of which the composition is determined in (c) ranges from 5-100.

17. Method for preparing a medicament for treating a disease, wherein the effective components as identified in accordance with any one of claims 1-16 are combined in the respective concentrations required for having an impact on the biological profile of the disease.

18. Method according to claim 17, wherein the effective components are synthetically prepared.

19. Method for controlling the composition of a multicomponent mixture, wherein the concentrations of at least two components of the mixture are adjusted to ensure that the at least two components of the mixture have an impact on a biological profile of the disease.

20. Method according to claim 19, wherein the multicomponent mixture comprises a natural product.
